# EUROPEAN PATENT SPECIFICATION

(11) **EP 1 796 725 B1**
(45) Date of publication and mention of the grant of the patent: **23.07.2008**
(21) Application number: 04774969.2
(22) Date of filing: 27.09.2004
(51) Int. Cl.: A61K 45/06, A61K 38/55, A61K 31/565

(54) **TREATMENT OR PREVENTION OF UNSCHELUDED BLEEDING IN WOMEN ON PROGESTOGEN CONTAINING MEDICATION**
BEHANDLUNG ODER PRÄVENTION VON UNPLANMÄSSIGEN BLUTUNGEN BEI FRAUEN UNTER PROGESTOGENHALTIGER MEDIKATION
TRAITEMENT OU PREVENTION DES HEMORRAGIES OCCASIONNELLES CHEZ LES FEMMES SOUS PROGESTOGENOTHERAPIE

(43) Date of publication of application: 20.06.2007
(73) Proprietor: Pantarhei Bioscience B.V., 3701 CH Zeist (NL)
(72) Inventor: NOTELOVITZ, Morris, Boca Raton, FL 33496 (US); COELINGH BENNINK, Herman, Jan, Tijmen, NL-2202 DA Noordwijk (NL); BOERRIGTER, Petrus, Jacobus, NL-7316 CP Apeldoorn (NL)
(74) Representative: Swinkels, Bart Willem
(86) International application number: PCT/NL2004/000667
(87) International publication number: WO 2006/036054

(56) References cited:
- DATABASE MEDLINE [Online] US NATIONAL LIBRARY OF MEDICINE (NLM), BETHESDA, MD, US; March 1997 (1997-03), PANAY N ET AL: "Progestogen intolerance and compliance with hormone replacement therapy in menopausal women." XP002254417 Database accession no. NLM9286739 & HUMAN REPRODUCTION UPDATE. ENGLAND 1997 MAR-APR, vol. 3, no. 2, March 1997 (1997-03), pages 159-171, ISSN: 1355-4786
- DATABASE MEDLINE [Online] US NATIONAL LIBRARY OF MEDICINE (NLM), BETHESDA, MD, US; January 1999 (1999-01), RIBSTEIN J ET AL: "Renal characteristics and effect of angiotensin suppression in oral contraceptive users." XP002254418 Database accession no. NLM9931087 & HYPERTENSION. UNITED STATES JAN 1999, vol. 33, no. 1, January 1999 (1999-01), pages 90-95, ISSN: 0194-911X
- DATABASE MEDLINE [Online] US NATIONAL LIBRARY OF MEDICINE (NLM), BETHESDA, MD, US; October 1979 (1979-10), OGIHARA T ET AL: "Effects of an angiotensin II antagonist;[sarcosine 1, isoleucine 8] angiotensin II, on blood pressure, plasma renin activity and plasma aldosterone concentration in hypertensive and normotensive subjects taking oral contraceptives." XP002254419 Database accession no. NLM393505 & ENDOCRINOLOGIA JAPONICA. JAPAN OCT 1979, vol. 26, no. 5, October 1979 (1979-10), pages 591-597, ISSN: 0013-7219
- DATABASE MEDLINE [Online] US NATIONAL LIBRARY OF MEDICINE (NLM), BETHESDA, MD, US; September 1994 (1994-09), BYRNE K B ET AL: "Effect of contraceptive steroid and enalapril treatment of systolic blood pressure and plasma renin-angiotensin in the rat." XP002254420 Database accession no. NLM7951167 & CLINICAL AND EXPERIMENTAL HYPERTENSION (NEW YORK, N.Y.: 1993) UNITED STATES SEP 1994, vol. 16, no. 5, September 1994 (1994-09), pages 627-657, ISSN: 1064-1963

## Description

### FIELD OF THE INVENTION

The present invention relates to the use of an effective amount of Renin Angiotensin System (RAS) suppressor selected from the group consisting of angiotensin converting enzyme inhibitors; angiotensin II receptor antagonists; renin inhibitors and combinations thereof for preparing a medicament for treating or preventing unscheduled bleeding (breakthrough bleeding or spotting) in women, said unscheduled bleeding being the result of repeated administration of a hormonal composition that contains a progestogen.

The invention further pertains to compositions and kits comprising the same RAS suppressor.

### BACKGROUND OF THE INVENTION

The repeated administration of a progestogen to women, e.g. as part of a contraceptive protocol or hormone replacement therapy, is known to give rise to unscheduled bleeding. Unscheduled bleeding can be defined as undesirable, unpredictable, irregular bleeding, which distinguishes this type of bleeding from the scheduled or regular withdrawal bleeding which is associated with the normal use of hormonal contraceptives as well as sequential regimens of hormone replacement therapy in peri- and postmenopausal women. Unscheduled bleeding may vary in intensity and is differentiated in spotting and breakthrough bleeding. Spotting is defined as slight unscheduled vaginal bleeding that usually requires at the most one sanitary pad or tampon a day, whereas breakthrough bleeding is defined as more pronounced unscheduled vaginal bleeding that requires more than one sanitary pad or tampon a day.

Unscheduled bleeding is a common complaint of oral contraceptive users. The failure to control the cycle for unscheduled bleeding episodes causes many women to stop using contraception and has an adverse affect on user compliance. It is well recognised that the symptoms of poor cycle control influence the occurrence of unintended pregnancies by encouraging the cessation of oral contraceptive use by women who do not wish to become pregnant.

Postmenopausal symptoms in women are frequently treated by hormone replacement therapy (HRT). HRT, like most hormonal contraceptive methods, usually employs the combined administration of an estrogen and a progestogen. Various treatment regimens are currently being utilised. In women with an intact uterus, the most widely used HRT regimens are sequentially and continuously combined estrogen/progestogen regimens. In sequentially combined estrogen/progestogen regimens, the continuous administration of an estrogen is sequentially combined with a progestogen for 10 to 14 days of every 28 days, i.e. a cyclic treatment of estrogen only for 14 to 18 days followed by a combination of the same estrogen and a progestogen for 10 to 14 days. The endometrial lining of the uterus is being stimulated during the estrogen only phase leading to increased thickness. The addition of the progestogen to the estrogen in the combined phase results in cessation of stimulation and secretory transformation of the endometrium just as in a natural menstrual cycle. After withdrawal of the progestogen, the transformed endometrium will be shedded from the uterine cavity and removed vaginally resulting in a bleeding that is referred to as 'scheduled bleeding', or 'withdrawal bleeding'.

In continuously combined estrogen/progestogen regimens, the continuous administration of an estrogen is continuously combined with a progestogen, i.e. there is no cyclic treatment. The continuous administration of an estrogen and a progestogen is known to result in an atrophic endometrium, i.e. it does not result in stimulation of the endometrial lining of the uterus. As the progestogen is not withdrawn during treatment, no scheduled bleeding occurs with this regimen.

Typically, oral contraception is a cyclic, 28-days treatment with a combination of an estrogen and a progestogen for 21 followed by 7 days without pill intake (i.e. stop week). In some oral contraceptives, 7 inactive tablets (placebo) are provided to not interrupt tablet taking in an effort to improve compliance. In effect, this leads to a treatment free week. An atypical, as compared to the natural cycle, endometrial development occurs during oral contraception treatment, which is referred to as 'pill endometrium'. During the stop (treatment-free) week, this endometrium is shedded and removed vaginally leading to a scheduled bleeding also referred to as 'withdrawal bleeding', which is the result of discontinuation of (i.e. withdrawing) the medication.

It is important to note that all known HRT and OC regimens are associated to a varying degree with unscheduled bleeding.

The research in the field of hormonal contraceptives as well as hormone replacement therapy has been directed towards developing hormonal compositions that minimise the potential for complications and side-effects, while maintaining efficacy. As a result, the amount of estrogen and progestogen in formulations for hormonal contraception and hormone replacement therapy has progressively decreased over the years and current formulations employ very low doses of estrogen and progestogen. Although having fewer side-effects and complications, particularly oral contraceptive formulations comprising low amounts of estrogens and progestogens exhibit a high occurrence of unscheduled bleeding, especially formulations comprising low amounts of estrogen, such as formulations comprising less than the equivalent of about 30 µg ethinylestradiol.

Several methods for decreasing the occurrence of unscheduled bleeding in connection with the use of formulations comprising estrogen and progestogen have been proposed in the past. One such method comprises the administration of estrogen and progestogen according to a specific regimen (WO 01/93848). Another method comprises the administration of formulations with a special estrogen/progestogen ratio (US 5,552,394). Despite the improvements achieved in these methods, the occurrence of unscheduled bleeding is not completely eliminated. Therefore, there still remains a need for further improvement.

It is the objective of the present invention to provide a method for decreasing the occurrence of unscheduled bleeding in women as a result of the repeated administration of a progestogen, e.g. as part of a contraceptive protocol or in connection with hormone replacement therapy.

### SUMMARY OF THE INVENTION

The inventors have unexpectedly found that the aforementioned objective may be realised by administering an effective amount of an Renin Angiotensin System (RAS) suppressor selected from the group consisting of angiotensin converting enzyme inhibitors; angiotensin II receptor antagonists; renin inhibitors; and combinations thereof.

Although the inventors do not wish to be bound by theory, it is believed that the a forementioned RAS suppresors prevent or reduce angiogenesis and/or neovascularisation within the endometrium, resulting in substantially less unscheduled bleeding.

The aforementioned RAS suppressors are capable of suppressing the renin-angiotensin system. The renin-angiotensin system has been studied extensively over the years and has been associated with important physiological functions. Below, a schematic representation of the main pathways is provided.

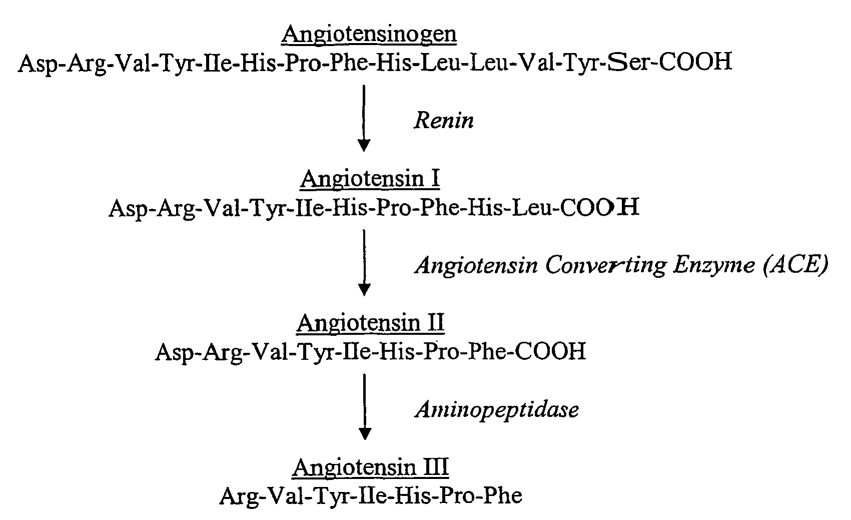

The central molecule in the renin-angiotensin system is angiotensin II, an octapeptide hormone, that is responsible for a wide range of effects including afferent and efferent vasoconstriction, aldosterone production and release, increased inotropism and chronotropism, proximal tubular re-adsorption of sodium, stimulation of drinking behaviour and sodium appetite, vagus suppression and β-adrenergic-receptor stimulation.

The initiating mechanism for increasing circulating angiotensin II levels is found in juxtaglomerular cells of the kidney. These cells secrete an aspartyl-protease, called renin, into the blood in response to decreases in arterial blood pressure, renal perfusion pressure and plasma sodium, or to increased sympathetic nervous activity. Renin acts on angiotensinogen, an α₂-globulin circulating in the plasma. Plasma angiotensinogen is primarily synthesised in the liver under the positive control of estrogens, glucocorticoids, thyroid hormones, and angiotensin II and is secreted through the constitutive pathway. Cleavage of the amino-terminal segment of angiotensinogen by renin releases a decapeptide prohormone, angiotensin I, which is further processed to the active octapeptide angiotensin II by the dipeptidyl carboxypeptidase called angiotensin-converting enzyme (ACE). Angiotensin II is further processed by an aminopeptidase to form the heptapeptide angiotensin III.

The known physiological and pharmacological actions of angiotensin II are effected through the binding of angiotensin II to angiotensin II receptors. These receptors can be classified into two subtypes, the angiotensin II-type 1 receptor and the angiotensin II-type 2 receptor. The former subtype is linked to the physiological and pharmacological actions of angiotensin II. Since most of these actions play a role in hypertension, cardiovascular diseases and cardiac failure, researchers have focussed on manipulating and blocking the effects of angiotensin II-type 1.

As regards the pharmacological manipulation of angiotensin II, three main categories may be distinguished: firstly, the use of inhibitors of renin, secondly, the use of inhibitors of ACE and, thirdly, the use of angiotensin receptor antagonists.

### DEFINITIONS

"Unscheduled bleeding" as used herein refers to any form of unpredictable bleeding as contrasted to the predictable withdrawal bleeding that is provoked by a sudden decrease in progestogen serum levels. "Treating" or "treatment" as used herein includes the reduction or the elimination of the occurrence of unscheduled bleeding (therapeutic treatment).

"Preventing" or "prevention" as used in relation to unscheduled bleeding refers to the prophylactic treatment of such bleeding, i.e. resulting in the reduction, delay or non-occurrence of unscheduled bleeding.

By "hormone replacement therapy" as used herein is meant the administration of female hormones to replace those no longer being produced naturally. Hormone replacement therapy may be used *inter alia* after the menopause or the surgical removal of the ovaries.

The term "antagonist" as used herein includes any molecule that partially or fully blocks, inhibits or neutralises a biological activity mediated by a receptor through preventing the binding of an agonist to the receptor (e.g. an angiotensin receptor), thereby blocking the biological activity of the agonist mediated by the receptor.

The term "inhibitor" as used in relation to a specific enzyme refers to a component that is capable of reducing or eliminating the *in vivo* activity of such an enzyme.

The term "therapeutically effective amount" or "effective amount" means that amount of a drug or a pharmaceutical or pharmacological active agent that is non-toxic, but will provide the desired effect.

The term "estrogen" as used throughout this document encompasses substances that are capable of triggering an estrogenic response *in vivo,* as well as precursors that are capable of liberating such an estrogen *in vivo* when used in accordance with the present invention. In order for estrogens to trigger such a response, they normally have to bind to an estrogen receptor.

The term "progestogen" is defined as a substance that is capable of triggering a progestogenic response *in vivo* or a precursor that is capable of liberating such a substance *in vivo*. Usually progestogens are capable of binding to a progestogen receptor.

The terms "angiotensin converting enzyme inhibitor"; "angiotensin II receptor antagonist"; "estrogen" and "progestogen" have a well understood meaning to persons skilled in the pharmaceutical or medical art. Descriptions of these pharmaceutically active substances and lists of representatives can, for instance, be found in Martindale, The complete drug reference. Thirty-third edition (2002); Goodman & Gilman's The Pharmacological basis of therapeutics., Tenth edition (2001); and Therapeutic Drugs, Second edition (1999).

A "pharmaceutically acceptable excipient" is a carrier material or combination of carrier materials that does not exhibit pharmaceutical activity and is generally safe and non-toxic.

### DETAILED DESCRIPTION OF THE INVENTION

One aspect of the present invention relates to the use of a RAS suppressor selected from the group consisting of angiotensin converting enzyme inhibitors; angiotensin II receptor antagonists; renin inhibitors; and combinations thereof for the manufacture of a medicament for use in a method of treating or preventing unscheduled bleeding in a woman, said unscheduled bleeding being the result of repeated administration of a hormonal composition that contains a progestogen, wherein the method comprises the administration of the RAS suppressor to the woman in an effective amount to reduce or prevent the incidence of unscheduled bleeding.

ACE-inhibitors that can be used in the method according to the invention include, but are not limited to, alacepril, benazepril, captopril, cilazapril, delapril, enalapril, enalaprilat, fosinopril, imidapril, lisinopril, perindopril, quinapril, fornopril, monopril, benazepril, ramipril, spirapril, moexipril, trandolapril,, pimobedan, zofenopril, temocapril, teprotide, trandalopril, zofenopril and N-[8-amino-1(S)-carboxyoctyl]-L-alanyl-L-proline. The term "ACE-inhibitor" also encompasses so called vasopeptidase inhibitors that are capable of simultaneously inhibiting both neutral endopeptidases and angiotensin-converting enzyme. Examples of vasopeptidase inhibitors include omapatrilat, sampatrilat, gemopatrilat, fasidotril, MDL-100240 and Z-13752A.

Angiotensin II receptor antagonists that can be used in the method according to the invention include, but are not limited to, saralasin, alsartan, irbesartan, candesartan, eprosartan, telmisartan, tasosartan, losartan, valsartan, olmesartan, pratosartan, PD 123177, CGP 42112A, BIBS 39, BIBS 222, Sar-Ile-Ang II, Sar-Thr-Ang II, Sar-Ala-Ang II, Sar-Val-Ala-Ang II, Sar-O-Me-Tyr-Ang II.

Renin inhibitors that can be used in the method according to the invention include, but are not limited to, aliskiren, remikiren, [N-(pyridyl-3-propionyl)-phenylalanyl-histidyl-(3S,4S) ACHPA-isoleucylamino]-2-methyl-2-dihydroxy-1,3-propane, Cl-992 and non-peptide renin inhibitors containing 2-(((3-phenylpropyl)phosphoryl)oxy)alkanoic acid moieties as P₂-P₃ replacements.

In a preferred embodiment of the present invention the RAS suppressor is selected from the group consisting of ACE inhibitors; angiotensin II receptor antagonists; and combinations thereof. Even more preferably the RAS suppressor is an angiotensin II-receptor antagonist.

The present use is particularly effective in women who suffer from unscheduled bleeding as a result of repeated administration of a hormonal composition that contains a combination of a progestogen and an estrogen. As explained herein before, such combinations are commonly applied in hormonal contraceptives and pharmaceutical compositions for use in hormone replacement therapy in peri- and postmenopausal women.

Typically, the estrogen is administered in an amount that is equivalent to a daily oral dosage of 3-50 µg, preferably of 5-40 µg ethinylestradiol. The progestogen is suitably administered in an amount that is equivalent to a daily oral dosage of 30-750 µg levonorgestrel, preferably of 50-400 µg levonorgestrel.

In a particularly preferred embodiment of the invention, the present use comprises the combined administration of the RAS suppressor and the hormonal composition. The combined administration may be achieved by administration within a short time interval (e.g. within 2 minutes) of separate dosage units that contain the individual pharmaceutically active components, or, alternatively, through administration of one or more dosage units that each contain the complete combination of pharmaceutically active components.

The present use may advantageously be used to treat women who suffer from unscheduled bleeding as a result of using a hormonal contraceptive or who are at risk of suffering from such unscheduled bleeding. Consequently, one embodiment of the invention is concerned with a method wherein the unscheduled bleeding is the result of repeated administration of the progestogen containing hormonal composition to a woman of childbearing capability in an effective amount to prevent ovulation.

An alternative embodiment of the invention is useful for the treatment or prevention of unscheduled bleeding in women who undergo hormone replacement therapy. In such an embodiment, the unscheduled bleeding is the result of repeated administration of the hormonal composition to a perimenopausal, menopausal or postmenopausal woman in an effective amount to prevent or suppress symptoms of hypoestrogenism. Hypoestrogenism can lead to disorders and ailments such as osteoporosis (loss of bone mass), arteriosclerosis and climacteric symptoms such as hot flushes (flashes), sweats, urogenital atrophy, mood disturbances, insomnia, palpitations.

The present invention may also suitably be used to treat or prevent unscheduled bleeding in women who undergo hormonal treatment of a gynaecological disorder, in particular a gynaecological disorder selected from the group consisting of endometriosis, uterine fibroids and premenstrual syndrome.

In order for the invention to be effective, it is advisable to continue the uninterrupted administration of the RAS suppressor for at least 1 month, preferably of at least 3 months, most preferably for at least 6 months. The benefits of the present invention are particularly pronounced if also the progestogen is administered concurrently with the RAS suppressor and uninterruptedly for at least 1 month, preferably at least 3 months and most preferably at least 6 months. The term "uninterrupted" as used in here, means that the pharmaceutically active component is administered at relatively regular intervals, with no (therapeutically) significant interruptions. Naturally, minor interruptions may occur that do not affect the overall effectiveness of the present method, and indeed such aberrations are encompassed by the present invention. In a preferred embodiment, and more arithmetically, the administration regimen is deemed to be uninterrupted if the longest interval between 2 subsequent administrations is not more than 3.5 times as long as the average interval. Even more preferably said longest interval is not more than 2.5 times, most preferably not more than 1.5 times as long as the average interval.

The effective amount of the RAS suppressor will depend on a number of factors including but not limited to bodyweight, age, the protocol for administration of the hormonal composition etc. In general, the use according to the invention comprises administering angiotensin II receptor antagonists in an amount that is equivalent to a daily oral dosage of at least 5 mg irbesartan. Preferably the administered dosage is equivalent to a daily oral dosage of at least 10 mg irbesartan, more preferably of at least 20 irbesartan. Usually the administered amount of angiotensin II receptor antagonists will not exceed the equivalent of a daily oral dosage of 150 mg irbesartan. Preferably the administered amount does not exceed the equivalent of a daily oral dosage of 100 mg irbesartan. More preferably said amount does not exceed the equivalent of a daily oral dosage 75 mg irbesartan, most preferably of a daily oral dosage of 50 mg.

Similarly, in the present invention, ACE inhibitor is usually administered in an amout equivalent to a daily oral dosage of at least 1 mg captopril, preferably of at least 2 and most preferably of at least 3 mg per day. Typically, the administered amount of ACE inhibitor does not exceed the equivalent of a daily oral dosage of 30 mg, preferably of 20 mg and most preferably of 15 mg.

As explained herein before, the RAS suppressor is employed in the present invention to suppress the effect of angiotensin II on angiogenesis and/or neovascularisation within the endometrium. This may suitably be achieved by inhibiting the endogenous production of angiotensin II through administration of an adequate amount of an ACE inhibitor and/or a renin inhibitor. Alternatively, this may also suitably be achieved by inhibiting the pharmacological action of angiotensin II not inhibiting the endogenous production but rather competitively occupying the specific receptor(s) without activating them through an angiotensin-receptor antagonist.

The RAS suppressor employed in the present invention may suitably be administered in any pharmaceutically acceptable way known in the art. Preferably said RAS suppressor is administered orally, intra-uterinely, intravaginally, transdermally or subcutaneously (e.g. as an implant). More preferably the RAS suppressor is administered orally or intra-uterinely, most preferably orally.

The hormonal composition may be administered enterally or parenterally. Preferably the hormonal composition is administered orally, transdermally, intra-uterinely, intravaginally, subcutaneously (e.g. as an implant) or intranasally. More preferably the hormonal composition is administered orally or transdermally, most preferably it is administered orally.

In a particularly preferred embodiment the RAS suppressor and the hormonal composition are administered in the same way, preferably orally, intra-uterinely or as a subcutaneous implant, most preferably orally.

Another aspect of the invention relates to a pharmaceutical composition containing at least 5 µg RAS suppressor; a progestogen in an amount that is equivalent to a daily oral dosage of at least 30 µg levonorgestrel; and a pharmaceutically acceptable excipient. Such a pharmaceutical composition may advantageously be employed in a so called progestogen-only contraceptive method, as the co-administration of the RAS suppressor will reduce the incidence of unscheduled bleeding in such a method. Preferably, the composition contains the RAS suppressor in an amount that is equivalent to a daily oral dose of at least 20 mg irbesartan. Typically, the aforementioned amount is equivalent to a daily oral dose of not more than 100 mg irbesartan, preferably of not more than 75 mg irbesartan and most preferably of not more than 50 mg irbesartan.

In a particularly preferred embodiment, the pharmaceutical composition additionally contains an estrogen in an amount that is equivalent to a daily oral dosage of at least 5 µg ethinylestradiol. As explained herein before, combinations ofprogestogenic and estrogens are commonly employed in contraceptive methods and HRT. The incorporation of the RAS suppressor in a pharmaceutical composition together with a progestogen and an estrogen offers the advantage that the resulting pharmaceutical composition gives less rise to unscheduled bleeding than do similar compositions that do not contain a RAS suppressor.

Orally administrable progestogens, estrogens and RAS suppressors are known in the art. Since oral administration is particularly convenient, the pharmaceutical composition of the present invention preferably is a solid unit dosage form for oral administration. Such a unit dosage form may suitably take the form of a pill, a tablet, a troche, a lozenge, a dispersible powder or granule, a hard capsule or a soft gelatin capsule.

Yet another aspect of the invention relates to a pharmaceutical kit comprising a plurality of dosage units, wherein at least one dosage unit contains a progestogen in an amount that is equivalent to a daily oral dosage of at least 30 µg levonorgestrel; at least one dosage unit contains an estrogen in an amount that is equivalent to a daily oral dosage of at least 5 µg ethinylestradiol ; and at least one dosage unit contains at least 5 µg of the RAS suppressor as defined herein before. Preferably, the dosage unit(s) containing the RAS suppressor comprise said suppressor in an amount that is equivalent to a daily oral dose of at least 20 mg irbesartan.

Examples of progestogens that may be employed in the present kit as well as in the method described herein before, include progesterone, levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagestone, chlormadinone, cyproterone acetate, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone, megestrol, melengestrol, nomegestrol, norethindrone (=norethisterone), norethynodrel, norgestrel (includes d-norgestrel and dl-norgestrel), norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, precursors of these compounds that are capable of liberating these progestogens *in vivo* when used in the present method and combinations thereof. Preferably the progestogen is selected from the group consisting of progesterone, desogestrel, etonogestrel, gestodene, dienogest, levonorgestrel, norgestimate, norethisterone, drospirenone, trimegestone, dydrogesterone, precursors of these progestogens and combinations thereof.

Examples of estrogens that may suitably be used in accordance with the present invention include ethinylestradiol, mestranol, quinestranol, estradiol, estrone, estran, estriol, estetrol, conjugated equine estrogens, precursors thereof that are capable of releasing such an estrogen *in vivo* when used in the present method and combinations thereof. Preferably the estrogen is selected from the group consisting of ethinylestradiol, estradiol, estetrol and combinations thereof.

The present invention may suitably employ esters of the progestogens and estrogens listed above. Such esters are capable of liberating an estrogen or a progestogen when used in the present method, e.g. as a result of metabolic conversion. Examples of suitable esters of estrogens and progestogens include such substances wherein the hydrogen atom of at least one of the hydroxyl groups has been substituted by an acyl radical of a hydrocarbon carboxylic, sulfonic acid or sulfamic acid of 1-25 carbon atoms; tetrahydrofuranyl; tetrahydropyranal; or a straight or branched chain glycosidic residue containing 1-20 glycosidic units per residue.

Typical examples of esters which can suitably be used in accordance with the invention are esters that can be obtained by reacting the hydroxyl groups of an estrogenic or progestogenic substance with substances that contain one or more carboxy (M^{+ -}OOC-) groups, wherein M⁺ represents a hydrogen or (akali)metal cation. Hence, in a particularly preferred embodiment, the esters are derivatives of one of the above listed estrogens or progestogens, wherein the hydrogen atom of at least one of the hydroxyl groups has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

For reasons of convenience and efficacy, it is preferred to combine.the aforementioned active principles in a single dosage unit such that the kit comprises a plurality of dosage units that contain a progestogen, an estrogen and a RAS suppressor. It is noted that not necessarily all the dosage units in the kit will contain these 3 components. Both in contraceptive and HRT regimens, it is feasible to administer a progestogen without an estrogen and *vice versa* during restricted intervals. Furthermore, it is common to employ intervals during which neither a progestogen or an estrogen are administered. Consequently, the present kit in addition to dosage units containing a progestogen, an estrogen and a RAS suppressor may contain dosage units that contain none of these active principles (e.g. placebo's), any one of these active principles or any possible combination of two of these active principles. Preferably the present kit comprises at least 10 dosage units that contain a progestogen, an estrogen and a RAS suppressor

The invention is further illustrated by means of the following examples.

### EXAMPLES

### Example 1

A clinical study is conducted in 40 healthy women of childbearing age. Two groups of 20 women who are not using oral contraception, 'pill starters', are enrolled. All subjects receive a low-dose oral contraceptive containing 20 µg ethinylestradiol and 100 µg levonorgestrel for 84 days. Twenty (20) subjects are randomized to additionally receive 75 mg irbesartan as an angiotensin II-receptor antagonist concomitantly with the oral contraceptive during the entire study period. All subjects document any unscheduled vaginal bleeding (spotting, breakthrough bleeding) in a diary. Blood pressure is monitored intensively.

Results show that women receiving angiotensin-II receptor antagonist treatment have fewer episodes of unscheduled bleeding and less intense blood loss, if any, as compared to the group receiving the oral contraceptive alone. The blood pressure pattern between groups does not differ significantly. The overall safety profile of the group receiving the combination of angiotensin II-receptor antagonist and oral contraceptive is comparable to that of the group receiving the oral contraceptive alone.

### Example 2

A clinical study is conducted in 40 healthy women of childbearing age. Two groups of 20 women who are not using oral contraception, 'pill starters', are enrolled. All subjects receive a progestogen-only oral contraceptive containing 75 µg desogestrel for 84 days. Twenty (20) subjects are randomized to additionally receive 50 mg irbesartan as an angiotensin II-receptor antagonist concomitantly with the oral contraceptive during the entire study period. All subjects document any unscheduled vaginal bleeding (spotting, breakthrough bleeding) in a diary. Blood pressure is monitored intensively.

Results show that women receiving angiotensin-II receptor antagonist treatment have fewer episodes of unscheduled bleeding and less intense blood loss, if any, as compared to the group receiving the oral contraceptive alone. The blood pressure pattern between groups does not differ significantly. The overall safety profile of the group receiving the combination of angiotensin II-receptor antagonist and oral contraceptive is comparable to that of the group receiving the oral contraceptive alone.

### Example 3

A clinical study is conducted in 40 healthy women of childbearing age. Two groups of 20 women are enrolled. All subjects receive two contraceptive injections containing 150 mg medroxyprogesterone acetate each with an interval of 3 months (13 weeks). Twenty (20) subjects are randomized to additionally receive daily 75 mg irbesartan as an angiotensin II-receptor antagonist during the entire study period of 6 months. All subjects document any unscheduled vaginal bleeding (spotting, breakthrough bleeding) in a diary. Blood pressure is monitored intensively.

Results show that women receiving angiotensin-II receptor antagonist treatment have fewer episodes of unscheduled bleeding and less intense blood loss, if any, as compared to the group receiving the contraceptive injection alone. The blood pressure pattern between groups does not differ significantly. The overall safety profile of the group receiving the combination of angiotensin II-receptor antagonist and contraceptive injection is comparable to that of the group receiving the contraceptive injection alone.

### Example 4

A clinical study is conducted in 40 healthy women of childbearing age. Two groups of 20 women are enrolled. All subjects receive an implant containing 68 mg etonogestrel. Twenty (20) subjects are randomized to additionally receive 75 mg irbesartan as an angiotensin II-receptor antagonist concomitantly with the contraceptive implant during the entire study period of 6 months. All subjects document any unscheduled vaginal bleeding (spotting, breakthrough bleeding) in a diary. Blood pressure is monitored intensively.

Results show that women receiving angiotensin-II receptor antagonist treatment have fewer episodes of unscheduled bleeding and less intense blood loss, if any, as compared to the group receiving the contraceptive implant alone. The blood pressure pattern between groups does not differ significantly. The overall safety profile of the group receiving the combination of angiotensin II-receptor antagonist and contraceptive implant is comparable to that of the group receiving the contraceptive implant alone.

### Example 5

A clinical study is conducted in 50 healthy women of childbearing age. Two groups of 25 women who are not using oral contraception, 'pill starters', are enrolled. All subjects receive a low-dose oral contraceptive containing 30 µg ethinylestradiol and 100 µg levonorgestrel for 84 days . Twenty-five (25) subjects are randomized to additionally receive 5 mg enalapril as an ACE-inhibitor with the oral contraceptive during the entire study period. All subjects document any unscheduled vaginal bleeding (spotting, breakthrough bleeding) in a diary. Blood pressure is monitored intensively.
Results show that women receiving the ACE-inhibitor treatment have fewer episodes of unscheduled bleeding and less intense blood loss, if any, as compared to the group receiving the oral contraceptive alone. The blood pressure pattern between groups does not differ significantly. The overall safety profile of the group receiving the combination of the ACE-inhibitor and oral contraceptive is comparable to that of the group receiving the oral contraceptive alone.

### Example 6

A clinical study is conducted in 40 healthy postmenopausal women, postmenopausal being defined as having had the last menstrual period at least 6 months before along with an increased FSH serum level. Two groups of 20 women who are not using any HRT are enrolled. All subjects receive a sequentially combined HRT preparation for 3 cycles of 28 days each. One cycle comprises 14 days of 2 mg estradiol only followed by 14 days of 2 mg estradiol for 14 days combined with 10 mg dydrogesterone. Twenty (20) subjects are randomized to additionally receive 75 mg irbesartan as an angiotensin II-receptor antagonist concomitantly with the HRT preparation during the entire study period. Subjects document any unscheduled vaginal bleeding (spotting, breakthrough bleeding) in a diary. Blood pressure is monitored intensively.

Results show that women receiving angiotensin-II receptor antagonist treatment have fewer episodes of unscheduled bleeding and less intense blood loss, if any, as compared to the group receiving the HRT preparation alone. Efficacy and blood pressure pattern between groups do not differ. The overall safety profile of the group receiving the combination of angiotensin II-receptor antagonist and HRT preparation is comparable to that of the group receiving the HRT preparation alone.

### Example 7

A clinical study is conducted in 40 healthy postmenopausal women, postmenopausal being defined as having had the last menstrual period at least 6 months before along with an increased FSH serum level. Two groups of 20 women who are not using any HRT are enrolled. All subjects receive a continuous combined HRT preparation containing 2 mg estradiol and 1 mg norethisterone acetate for 84 days. Twenty (20) subjects are randomized to additionally receive 50 mg irbesartan as an angiotensin II-receptor antagonist concomitantly with the HRT preparation during the entire study period. Subjects document any unscheduled vaginal bleeding (spotting, breakthrough bleeding) in a diary. Blood pressure is monitored intensively.
Results show that women receiving angiotensin-II receptor antagonist treatment have fewer episodes of unscheduled bleeding and less intense blood loss, if any, as compared to the group receiving the HRT preparation alone. Efficacy and blood pressure pattern between groups do not differ significantly. The overall safety profile of the group receiving the combination of angiotensin II-receptor antagonist and HRT preparation is comparable to that of the group receiving the HRT preparation alone.

### Example 8

A clinical study is conducted in 50 healthy postmenopausal women, postmenopausal being defined as having had the last menstrual period at least 6 months before along with an increased FSH serum level. Two groups of 25 women who are not using any HRT are enrolled. All subjects receive a continuous combined HRT preparation containing 1 mg estradiol and 2 mg drosperinone for three periods of 28 days each. Twenty-five (25) subjects are randomized to additionally receive 15 mg captopril, an ACE-inhibitor concomitantly with the HRT preparation during the entire study period. Subjects document any unscheduled vaginal bleeding (spotting, breakthrough bleeding) in a diary. Blood pressure is monitored intensively.

Results show that women receiving the ACE-inhibitor have fewer episodes of unscheduled bleeding and less intense blood loss, if any, as compared to the group receiving the HRT preparation alone. Efficacy and blood pressure pattern between groups do not differ significantly. The overall safety profile of the group receiving the combination of an ACE-inhibitor and HRT preparation is comparable to that of the group receiving the HRT preparation alone.

## Claims

1. Use of a Renin Angiotensin System (RAS) suppressor selected from the group consisting of angiotensin converting enzyme inhibitors (ACE-inhibitors); angiotensin II receptor antagonists; renin inhibitors; and combinations thereof in the manufacture of a medicament for use in a method of treating or preventing unscheduled bleeding in a woman, said unscheduled bleeding being the result of repeated administration of a hormonal composition that contains a progestogen, wherein said method comprises the administration of the RAS suppressor to the woman in an effective amount to reduce or prevent the incidence of unscheduled bleeding.

2. Use according to claim 1, wherein the hormonal composition also contains an estrogen.

3. Use according to claim 1 or 2, wherein the RAS suppressor is selected from the group consisting of ACE-inhibitors; angiotensin II receptor antagonists; and combinations thereof.

4. Use according to any one of claims 1-3, wherein the method comprises the combined administration of the RAS suppressor and the hormonal composition.

5. Use according to any of the claims 1-4, wherein the unscheduled bleeding is the result of repeated administration of the hormonal composition to a woman of childbearing capability in an effective amount to prevent ovulation.

6. Use according to any one of claims 2-4, wherein the unscheduled bleeding is the result of repeated administration of the hormonal composition to a perimenopausal, menopausal or postmenopausal woman in an effective amount to prevent or suppress symptoms of hypoestrogenism.

7. Use according to any of the claims 1-6, wherein the method comprises the uninterrupted administration of the RAS suppressor during a period of at least 1 month, preferably of at least 3 months.

8. Use according to any of the claims 1-7, wherein the method comprises administering angiotensin II receptor antagonist in an amount equivalent to a daily oral dosage of at least 5 mg irbesartan.

9. Use according to claim 8, wherein the method comprises administering ACE inhibitor in an amount equivalent to a daily oral dosage of at least 1 mg captopril.

10. Use according to any one of claims 1-9, wherein the RAS suppressor is administered orally or intra-uterinely.

11. Use according to any one of claims 1-10, wherein the hormonal composition is administered orally, intra-uterinely or transdermally.

12. A pharmaceutical composition containing at least 5 µg RAS suppressor, said RAS suppressor being selected from the group consisting of ACE inhibitors, angiotensin II receptor antagonists and renin inhibitors; a progestogen in an amount equivalent to at least 30 µg levonorgestrel; and pharmaceutically acceptable excipient.

13. The pharmaceutical composition according to claim 12, wherein the composition additionally contains an estrogen in an amount equivalent to at least 5 µg ethinylestradiol.

14. The pharmaceutical composition according to claim 12 or 13, wherein the composition is a solid unit dosage form for oral administration.

15. A pharmaceutical kit comprising a plurality of dosage units, wherein at least one dosage unit contains a progestogen in an amount equivalent to at least 30 µg levonorgestrel; at least one dosage unit contains an estrogen in an amount equivalent to 5 µg ethinyl estradiol; and at least one dosage unit contains a RAS suppressor selected from the group consisting of ACE inhibitors, angiotensin II receptor antagonists, renin inhibitors and combinations thereof.

16. The kit according to claim 15, wherein the kit comprises a plurality of dosage units that contain a progestogen, an estrogen and a RAS suppressor.

## Patentansprüche

1. Verwendung eines Renin-Angiotensin-System(RAS)-Suppressors, der aus der Gruppe ausgewählt ist, bestehend aus Angiotensin-Umwandlungsenzym-Inhibitoren (ACE-Inhibitoren); Antiotensin-II-Rezeptor-Antagonisten; Renin-Inhibitoren und Kombinationen davon, bei der Herstellung eines Medikaments zur Verwendung bei einem Verfahren zur Behandlung oder Prävention von unplanmäßigen Blutungen bei einer Frau, wobei die unplanmäßigen Blutungen das Ergebnis wiederholter Verabreichung einer Hormon-Zusammensetzung sind, die ein Progestagen enthält, wobei das Verfahren die Verabreichung des RAS-Suppressors an die Frau in einer wirksamen Menge zur Herabsetzung oder Prävention der Inzidenz unplanmäßiger Blutungen umfasst.

2. Verwendung nach Anspruch 1, wobei die Hormon-Zusammensetzung auch ein Östrogen enthält.

3. Verwendung nach Anspruch 1 oder 2, wobei der RAS-Suppressor aus der Gruppe ausgewählt ist, bestehend aus ACE-Inhibitoren; Antiotensin-II-Rezeptor-Antagonisten und Kombinationen davon.

4. Verwendung nach einem der Ansprüche 1 bis 3, wobei das Verfahren die kombinierte Verabreichung des RAS-Suppressors und der Hormon-Zusammensetzung umfasst.

5. Verwendung nach einem der Ansprüche 1 bis 4, wobei die unplanmäßige n Blutungen das Ergebnis wiederholter Verabreichung der Hormon-Zusammensetzung an eine gebärfähige Frau in einer wirksamen Menge zur Verhinderung der Ovulation sind.

6. Verwendung nach einem der Ansprüche 2 bis 4, wobei die unplanmäßigen Blutungen das Ergebnis wiederholter Verabreichung der Hormon-Zusammensetzung an eine perimenopausale, menopausale oder postmenopausale Frau in einer wirksamen Menge zur Verhinderung oder Unterdrückung von Symptomen des Hypoöstrogenismus sind.

7. Verwendung nach einem der Ansprüche 1 bis 6, wobei das Verfahren die ununterbrochene Verabreichung des RAS-Suppressors während eines Zeitraums von mindestens 1 Monat, vorzugsweise von mindestens 3 Monaten umfasst.

8. Verwendung nach einem der Ansprüche 1 bis 7, wobei das Verfahren die Verabreichung eines Angiotensin-II-Rezeptor-Antagonisten in einer Menge entsprechend einer täglichen oralen Dosis von mindestens 5 mg Irbesartan umfasst.

9. Verwendung nach Anspruch 8, wobei das Verfahren die Verabreichung von ACE-Inhibitor in einer Menge entsprechend einer täglichen oralen Dosis von mindestens 1 mg Captopril umfasst.

10. Verwendung nach einem der Ansprüche 1 bis 9, wobei der RAS-Suppressor oral oder intrauterin verabreicht wird.

11. Verwendung nach einem der Ansprüche 1 bis 10, wobei die Hormon-Zusammensetzung oral, intrauterin oder transdermal verabreicht wird.

12. Pharmazeutische Zusammensetzung, die mindestens 5 µg RAS-Suppressor, wobei der RAS-Suppressor aus der Gruppe ausgewählt ist, bestehend aus ACE-Inhibitoren, Angiotensin-II-Rezeptor-Antagonisten und Renin-Inhibitoren, ein Progestagen in einer Menge entsprechend mindestens 30 µg Levonorgestrel und einen pharmazeutisch verträglichen Exzipienten enthält.

13. Pharmazeutische Zusammensetzung nach Anspruch 12, wobei die Zusammensetzung zusätzlich ein Östrogen in einer Menge entsprechend mindestens 5 µg Ethinylestradiol enthält.

14. Pharmazeutische Zusammensetzung nach Anspruch 12 oder 13, wobei die Zusammensetzung eine feste Dosierungseinheitsform zur oralen Verabreichung ist.

15. Pharmazeutisches Kit umfassend eine Vielzahl von Dosierungseinheiten, wobei mindestens eine Dosierungseinheit ein Progestagen in einer Menge entsprechend mindestens 30 µg Levonorgestrel enthält; mindestens eine Dosierungseinheit ein Östrogen in einer Menge entsprechend 5 µg Ethinylestradiol enthält; und mindestens eine Dosierungseinheit einen RAS-Suppressor enthält, der aus der Gruppe ausgewählt ist, bestehend aus ACE-Inhibitoren, Angiotensin-II-Rezeptor-Antagonisten, Renin-Inhibitoren und Kombinationen davon.

16. Kit nach Anspruch 15, wobei das Kit eine Vielzahl von Dosierungseinheiten umfasst, die ein Progestagen, ein Östrogen und einen RAS-Suppressor enthalten.

## Revendications

1. Utilisation d'un suppresseur du Système Rénine Angiotensine (RAS) sélectionné dans le groupe constitué d'inhibiteurs de l'enzyme de conversion de l'angiotensine (inhibiteurs ACE); d'antagonistes du récepteur d'angiotensine II ; d'inhibiteurs de rénine ; et de leurs combinaisons pour la fabrication d'un médicament pour utilisation dans un procédé de traitement ou de prévention d'un saignement imprévu chez une femme, ce saignement imprévu étant le résultat d'une administration répétée d'une composition hormonale contenant un progestatif, le procédé comprenant l'administration du suppresseur du RAS à la femme dans une quantité efficace pour réduire ou prévenir l'incidence du saignement imprévu.

2. Utilisation selon la revendication 1, dans laquelle la composition hormonale comprend en outre un oestrogène.

3. Utilisation selon la revendication 1 ou 2, dans laquelle le suppresseur du RAS est sélectionné parmi le groupe constitués d'inhibiteurs ACE; d'antagonistes du récepteur d'angiotensine II ; et de leurs combinaisons.

4. Utilisation selon l'une quelconque des revendications 1 à 3, dans laquelle le procédé comprend l'administration combinée du suppresseur du RAS et de la composition hormonale.

5. Utilisation selon l'une quelconque des revendications 1 à 4, dans laquelle le saignement imprévu est le résultat d'une administration répétée de la composition hormonale à une femme capable d'avoir des enfants dans une quantité efficace pour prévenir une ovulation.

6. Utilisation selon l'une quelconque des revendications 2 à 4, dans laquelle le saignement imprévu est le résultat d'une administration répétée de la composition hormonale à une femme en périménopause, en ménopause ou en postménopause dans une quantité efficace pour prévenir ou supprimer des symptômes d' hypo-oestrogénisme.

7. Utilisation selon l'une quelconque des revendications 1 à 6, dans laquelle le procédé comprend l'administration ininterrompue du suppresseur du RAS durant une période d'au moins un mois, de préférence d'au moins 3 mois.

8. Utilisation selon l'une quelconque des revendications 1 à 7, dans laquelle le procédé comprend une administration d'antagoniste du récepteur d'angiotensine II dans une quantité équivalente à un dosage oral journalier d'au moins 5 mg d'irbésartan.

9. Utilisation selon la revendication 8, dans laquelle le procédé comprend une administration d'inhibiteur ACE dans une quantité équivalente à un dosage oral journalier d'au moins 1 mg de captopril.

10. Utilisation selon l'une quelconque des revendications 1 à 9, dans laquelle le suppresseur du RAS est administré oralement ou de façon intra-utérine.

11. Utilisation selon l'une quelconque des revendications 1 à 10, dans laquelle la composition hormonale est administrée oralement, de façon intra-utérine ou de façon transdermale.

12. Une composition pharmaceutique contenant au moins 5 µg de suppresseur du RAS, ledit suppresseur du RAS étant sélectionné dans le groupe constitué d'inhibiteurs ACE, d'antagonistes du récepteur d'angiotensine II et d'inhibiteurs de rénine ; un progestatif dans une quantité équivalente à au moins 30 µg de lévonorgestrel ; et un excipient acceptable pharmaceutiquement.

13. La composition pharmaceutique selon la revendication 12, dans laquelle la composition contient de façon additionnelle un oestrogène dans une quantité équivalente à au moins 5 µg d'éthinylestradiol.

14. La composition pharmaceutique selon la revendication 12 ou 13, dans laquelle la composition est de forme de dosage en unité solide pour une administration orale.

15. Un kit pharmaceutique comprenant une pluralité d'unités de dosage, dans lequel au moins une unité de dosage comprend un progestatif dans une quantité équivalente à au moins 30 µg de lévonorgestrel ; au moins une unité de dosage comprend un oestrogène dans une quantité équivalente à 5 µg d'éthinyl estradiol ; et au moins une unité de dosage comprend un suppresseur du RAS sélectionné dans le groupe constitué d'inhibiteurs ACE, d'antagonistes du récepteur d'angiotensine II, d'inhibiteurs de rénine et de leurs combinaisons.

16. Le kit selon la revendication 15, dans lequel le kit comprend une pluralité d'unités de dosage qui contiennent un progestatif, un oestrogène et un suppresseur du RAS.
